# EUROPEAN PATENT APPLICATION

(11) **EP 0 712 620 A1**
(43) Date of publication of application: **22.05.1996**
(21) Application number: 95200727.6
(22) Date of filing: 23.03.1995
(51) Int. Cl.: A61H 39/04

(54) **Magnetized quartz adjustable body belt**

(30) Priority: 18.11.1994 GB 9423320
(71) Applicant: Nisbet, John Stoddart Jeffrey, Douglas, Isle of Man IM2 4HA (GB)
(72) Inventor: Nisbet, John Stoddart Jeffrey, Douglas, Isle of Man IM2 4HA (GB)

(57) **Abstract**

The bodybelt (A) holds magnets (D) with quartz (E) or similar crystals in position on the body, so that a magnetic field is created allowing the healing power of the crystal to be effective.

The bodybelts can also be worn round neck (similar to chain of office). Provided is further a waistcoat with velcro lining so that pads with 2 or more magnets, with quartz or similar crystals attached, can be precisely positioned, since the pads are lined on one side with velcro.

The strength of the magnets may be varied, similarly the thickness of the slices of crystal. A huge number of different crystals can be employed.

## Description

### REFERRING TO DRAWING SHEET 1.

### Bodybelt

- Sketches 1 & 2.: Shows the bodybelt (A) with adjustable braces (B), which enable bodybelt to be supported at varying positions around the body. The encirclement of the body by this bodybelt (A) is completed using a velcro flap (C).
- Sections A-A & B-B.: Shows full size sections of the bodybelt (A) with magnet (D) with crystal slice (C) attached by means of an adhesive. Holes have been cut in felt (F) to accommodate magnet (D) with crystal (E) attached at 4" centres along the bodybelt (A). Nine magnets (D) with crystals (E) attached for a 36" waist measurement. Firm cotton fabric (G) is used as the external covering to which velcro (C) is attached at either end as a means of closing belt.
- Waistcoat: Using similar construction to bodybelt (A), pads with 2 or 3 magnets (D) with crystal slices (E) attached, but with velcro on one side, this allows the pads to be fixed to the inside of a waistcoat, which has been lined with velcro. This allows coverage of parts of the body not covered by the bodybelt (A).
- Knee, Hand, Ankle Supports.: Using similar methods magnets (D) with crystal slices (E) attached, knee, hand and ankle supports could be used to pinpoint areas for treatment. Other areas of concern could be similarly treated, i.e. thighs, upper arm, wrist etc.

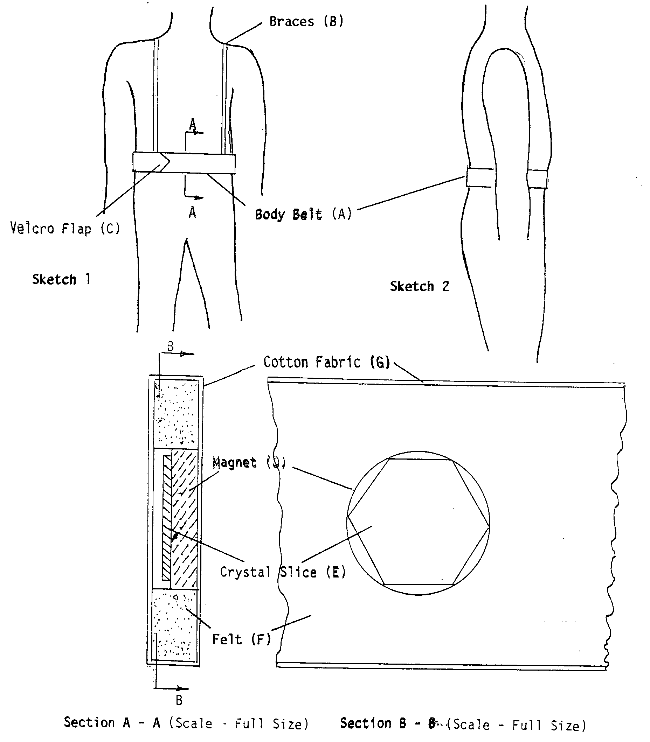

## Claims

1. That the magnetized quartz or similar crystal adjustable bodybelt, waistcoat, knee, hand and ankle support and other similar methods of applying the healing power of magnets with quartz or similar crystals,will give similar results when applied to one's body.
Letter - Isabel Marshall. Relief, using belt and waistcoat, from back and shoulder pains, also gets relaxed nights rest.
Letter - Rhoda Hanlon. Relief using waistcoat, from tortuous neck and back pains and gets relaxed nights rest.
Letter - James Douglas. Relief using belt and waistcoat, from chronic pains back and shoulders, also hypertension and high blood pressure is better due to calmness from use of belt and waistcoat, plus relaxed sleep.
Letter - Julian Glover. Relief using belt round neck, from tension in neck plus relaxed sleep.
Letter - Daphne Nisbet. Relief using belt, gives improved sleep and stress free life. (Wife of cancer victim).
Letter - John Nisbet. Relief using belt, reduces stress to a minimum. Mr. Nisbet suffers from cancer and has a short prognosis.
Letter - Nurse Hemmingsen. Relief using belt and waistcoat, from stress and premenstrual tension, also fibroadenitis (periodic thickening of breasts), which causes pain.
Letter - Mr. & Mrs.Quinn. Relief using belt from pain and stress - caused by Mr. Quinn's cancer.
Letter - Kathleen Ramsay. Relief using belt from stress and insomnia.
Letter - June Niven. Relief using hand strap for arthritis.
The foregoing abbreviated letters show in general that the magnets with quartz attached, when placed in position using bodybelts, waistcoats etc., that stress and pain are effectively dealt with. Trials using varying strengths of magnets and different types of crystals sliced in varying thickness's, will continue.
